(19) **Europäisches Patentamt**
**European Patent Office**
**Office européen des brevets**

(11) **EP 1 848 329 B1**

(12) **EUROPÄISCHE PATENTSCHRIFT**

(45) Veröffentlichungstag und Bekanntmachung des Hinweises auf die Patenterteilung:
**23.03.2011 Patentblatt 2011/12**

(51) Int Cl.:
***A61B 5/028*** *(2006.01)*  ***A61B 5/029*** *(2006.01)*

(21) Anmeldenummer: **06700675.9**

(22) Anmeldetag: **03.01.2006**

(86) Internationale Anmeldenummer:
**PCT/EP2006/050006**

(87) Internationale Veröffentlichungsnummer:
**WO 2006/087245 (24.08.2006 Gazette 2006/34)**

(54) **VORRICHTUNG ZUR BESTIMMUNG KARDIOPULMONALER VOLUMINA UND FLÜSSE EINES LEBEWESENS**

DEVICE FOR DETERMINING CARDIOPULMONARY VOLUMES AND FLOWS OF A LIVING BEING

DISPOSITIF DE DETERMINATION DE VOLUMES ET DE COURANTS CARDIOPULMONAIRES D'UN ETRE VIVANT

(84) Benannte Vertragsstaaten:
**DE ES FR GB IT**

(30) Priorität: **18.02.2005 DE 102005007592**

(43) Veröffentlichungstag der Anmeldung:
**31.10.2007 Patentblatt 2007/44**

(73) Patentinhaber: **Pulsion Medical Systems AG**
**81829 München (DE)**

(72) Erfinder:
- **PFEIFFER, Ulrich J.**
**81667 München (DE)**
- **KNOLL, Reinhold**
**81543 München (DE)**
- **MICHARD, Frédéric**
**Somerville, Massachusetts 02144 (US)**

(74) Vertreter: **Ettmayr, Andreas et al**
**Kehl & Ettmayr**
**Patentanwälte**
**Friedrich-Herschel-Straße 9**
**81679 München (DE)**

(56) Entgegenhaltungen:
**US-A- 5 595 181**

- **CUTLER C A ET AL: "A thermodilution method for quantification of bidirectional shunts." COMPUTERS AND BIOMEDICAL RESEARCH, AN INTERNATIONAL JOURNAL. AUG 1979, Bd. 12, Nr. 4, August 1979 (1979-08), Seiten 379-410, XP002373786 ISSN: 0010-4809**

**Beschreibung**

Die vorliegende Erfindung betrifft eine Vorrichtung zur Bestimmung mindestens eines hämodynamischen Parameters eines Lebewesens, insbesondere eine Vorrichtung zur Bestimmung kardiopulmonaler Volumina und Flüsse eines Lebewesens.

Vorrichtungen zur Bestimmung hämodynamischer Parameter aus einer mittels invasiver Messungen gewonnenen Dilutionskurve sind insbesondere in der Intensivmedizin weit verbreitet. Bei den hämodynamischen Parametern handelt es sich dabei vor allem um charakteristische Volumina bzw. Volumenströme, wie etwa das Herzeitvolumen (*Cardiac Output*, CO), das globale enddiastolische Volumen (GEDV) und das Volumen des extravasalen Lungenwassers (EVLW). Entsprechende Systeme sind kommerziell verfügbar und arbeiten meist mit Kälte (i.e. einem gekühlten Bolus) als Indikator. Neben den verbreiteten Rechtsherzkathetersystemen, mit welchen Thermodilutionsmessungen mit der Lungenarterie als Meßort durchgeführt werden, haben sich Systeme zur transpulmonalen Thermodilutionsmessung auf dem Markt etabliert.

Verfahren und Vorrichtungen zur transpulmonalen Thermodilutionsmessung sind unter anderem in WO 93/21823 A1 und WO 01/30237 A1 sowie darin genannter Literatur offenbart.

Bei der Bestimmung hämodynamischer Parameter anhand von gemessenen Dilutionskurven können aufgrund von patientenspezifischen Anomalien Ungenauigkeiten bzw. Fehler auftreten. Zu derartigen Anomalien gehören Kurzschlussströmungen vom rechten Atrium zum linken Atrium (sogenannter Rechts-Links-Shunt, RL-Shunt) bzw. vom linken Ventrikel zum rechten Ventrikel (sogenannter Links-Rechts-Shunt, LR-Shunt).

In US 5,595,181 wird die Bestimmung eines Links-Rechts-Shunts im Rahmen einer Thermodilutionsmessung mit Rechtsherzkatheter offenbart. Die Shunt-Bestimmung erfolgt dabei durch Vergleich des zeitlichen Temperaturverlaufs mit einem angenommenen Temperaturverlauf ohne Shunt. Da ein Temperaturverlauf ohne Shunt bei demselben Individuum unter identischen Bedingungen zwangsläufig nicht bekannt ist, handelt es sich hierbei lediglich um eine Schätzung eher geringer Genauigkeit. Die Verwendung eines Rechtsherzkatheters in Form eines herkömmlichen Ballonkatheters birgt zudem ein nicht unerhebliches medizinisches Risiko, da hier grundsätzlich das Herz selbst Objekt einer invasiven Massnahme ist. Ferner wird der wesentlich häufiger auftretende Defekt eines Rechts-Links-Shunts nicht berücksichtigt.

In der Dissertation J. K. G. Wietasch, "Die Doppelindikatordilution zur Quantifizierung von Herzzeitvolumen und Links-Rechts-Shunt bei Patienten mit kongenitalem Vitium cordis", Göttingen 1995 wird die Bestimmung eines Links-Rechts-Shunts mittels Doppelindikatordilutionstechnik beschrieben, d.h. durch parallele Ermittlung von Dilutionskurven mittels pulmonalarterieller und aortaler Messung. Auch hier ist für die pulmonalarterielle Messung die Applikation eines Rechtsherzkatheters mit den damit verbundenen medizinischen Risiken erforderlich.

Vor diesem Hintergrund ist es Aufgabe der Erfindung, eine Vorrichtung zur Bestimmung hämodynamischer Parameter eines Lebewesens zu schaffen, welche auch bei Patienten mit Kurzschlussströmungen verursachenden Herzfehlern eine zuverlässige, möglichst patientenschonende und weniger fehlerbehaftete hämodynamische Überwachung gewährleisten.

Gemäss einem Aspekt der vorliegenden Erfindung wird diese Aufgabe mit einer Vorrichtung nach Anspruch 1 gelöst.

Vorteilhafte Ausführungsformen der Erfindung können gemäss einem der Ansprüche 2-22 gestaltet sein.

Auf für den Fachmann überraschende Weise ist erfindungsgemäss eine geeignete programmtechnische Einrichtung der Auswerteeinheit einer transpulmonalen Messanordnung, vorzugsweise mit zentralvenösem und arteriellem Katheter, hinreichend, um eine mögliche Kurzschlussströmung von der rechten zur linken Herzhälfte (RL-Shunt) und/oder von der linken zur rechten Herzhälfte (LR-Shunt) des Lebewesens zu berücksichtigen, ohne dass hierbei der Einsatz eines Rechtsherzkatheters erforderlich wäre, oder überhaupt ein Rückgriff auf pulmonalarterielle Messwerte zu erfolgen hätte.

Dabei wird ein Modell zugrundegelegt, welches die der Dilutionskurve entsprechende Funktion y als Faltung der Störfunktion I mit mehreren, charakteristische Zeiten als Modellparameter enthaltenden Ausdrücken beinhaltet. Die Ausdrücke entsprechen ideal durchmischten Volumina bzw. Verzögerungsgliedern, welche vereinfachend für das rechte Atrium RA, den rechten Ventrikel RV, das pulmonale Blutvolumen PBV, das extravasale Thermovolumen ETV, das linke Atrium LA und den linken Ventrikel LV angesetzt werden.

Der Shunt kann in beiden Richtungen sowohl intrakardial als auch extrakardial sein.

Vorzugsweise ist die Auswerteeinheit programmtechnisch zur Durchführung der folgenden Schritte eingerichtet: (a) Abschätzen eines Startpunkts und eines Dilutions-Peaks der Dilutionskurve y, (b) Berechen einer mittleren Durchgangszeit $MTT = \int y \cdot t \, dt / \int y \, dt$ (mit Zeitvariable t) und einer Abklingzeit DST (aus dem exponentiellen Abfall der Dilutionskurve y gemäss $y \propto \exp(-t/DST)$ nach dem Dilutions-Peak, (c) Bestimmen von Modellparametern des zugrundegelegten Modells unter Verwendung der mittleren Durchgangszeit MTT und der Abklingzeit DST, (e) Berechnen des Herzeitvolumens CO und eines Kurzschlussströmungsverhältnisses s, (f) Berechnen der die Modellparameter enthaltenden Ausdrücke, und (g) Berechnen des hämodynamischen Parameters.

Das Bestimmen der Modellparametern kann vorteilhafterweise durch die Teilschritte (i) Anpassen einer Modellkurve an die Dilutionskurve (beispielsweise mittels eines Levenberg-Marquardt-Algorithmus), und (ii) Ermitteln der Modellparameter aus der Modellkurve erfolgen.

Alternativ können die Modellparametern vorteilhafterweise auch durch folgende Teilschritte bestimmt werden: (i) Ermitteln eines vor dem Dilutionspeak liegenden Kurzschluß-Peaks, (ii) Bestimmen einer Tangente an die Dilutionskurve unterhalb des Kurzschluß-Peaks, welche mit der Dilutionskurve die größtmögliche Fläche einschließt, und (iii) Abschätzen der Modellparameter mit Hilfe von Kurvenparametern welche aus der Lage des Startpunkts der Dilutionskurve, der Berührpunkte der Tangente, des Kurzschluß-Peaks und des Dilutions-Peaks bestimmbar sind.

Auch wenn gemäß einer bevorzugten Ausführungsform eine zentralvenöse und eine arterielle Kathetereinheit vorgesehen sind, können auch alternative Ausführungsformen der Erfindung vorteilhaft sein, bei welchen das arterielle Signal nichtinvasiv, beispielsweise über eine tympanometrische Temperaturmeßstelle oder mittels optischer Methoden, erfaßt wird, und/oder die Systemstörung nicht zentralvenös sondern peripher ausgelöst wird. Im letztgenannten Fall muß lediglich in hinreichender Näherung bekannt sein oder abgeschätzt werden können, welche zusätzliche Verzögerung durch die periphere Auslösung zu berücksichtigen ist.

Grundsätzlich kann die Störung durch Wärmezufuhr, "Kältezufuhr" (Injektion eines gekühlten Bolus), Lithiumchlorid-Injektion (LiCl), Indocyaningrün-Injektion (ICG) oder andere Indikatoren erfolgen.

Die Störfunktion kann prinzipiell einen beliebigen (aber mit hinreichender Genauigkeit bekannten) Verlauf haben, beispielsweise ist auch eine pseudostochastische Verteilung möglich.

Grundsätzlich kann jede im Rahmen der vorliegenden Anmeldung beschriebene bzw. angedeutete Variante der Erfindung besonders vorteilhaft sein, je nach wirtschaftlichen und technischen Bedingungen im Einzelfall. Soweit nichts gegenteiliges dargelegt ist, bzw. soweit grundsätzlich technisch realisierbar, sind einzelne Merkmale der beschriebenen Ausführungsformen austauschbar oder miteinander kombinierbar.

Nachfolgend werden anhand der zugehörigen Zeichnungen Beispiele bevorzugter Ausführungsformen der vorliegenden Erfindung näher erläutert. Die Zeichnungen sind dabei rein schematisch aufzufassen. Es zeigt:

Fig. 1    eine schematisch skizzenhafte Darstellung eines Herz-Kreislaufsystems mit der Anordnung wesentlicher Komponenten einer erfindungsgemäßen Vorrichtung,

Fig. 2    eine schaltbildartige Veranschaulichung möglicher Kurzschlußströmungen

Fig. 3    eine schaltbildartige Skizze zur Berücksichtigung eines Rechts-Links-Shunts gemäß einem erfindungsgemäß zugrundelegbaren Modell,

Fig. 4    eine skizzenhafte Darstellung einer Dilutionskurve y(t) mit einem auf Rechts-Links-Shunt zurückgehenden Peak vor dem Dilutionspeak,

Fig. 5    eine schaltbildartige Skizze zur Berücksichtigung eines Links-Rechts-Shunts gemäß einem erfindungsgemäß zugrundelegbaren Modell,

Fig. 6    eine skizzenhafte Darstellung einer Dilutionskurve y(t) mit einer auf Links- Rechts-Shunt zurückgehenden Abflachung hinter dem Dilutionspeak, sowie

Fig. 7    eine schaltbildartige Skizze zur Berücksichtigung von Links-Rechts-Shunt, Rechts-Links-Shunt und Rezirkulation gemäß einem erfindungsgemäß zugrundelegbaren Modell.

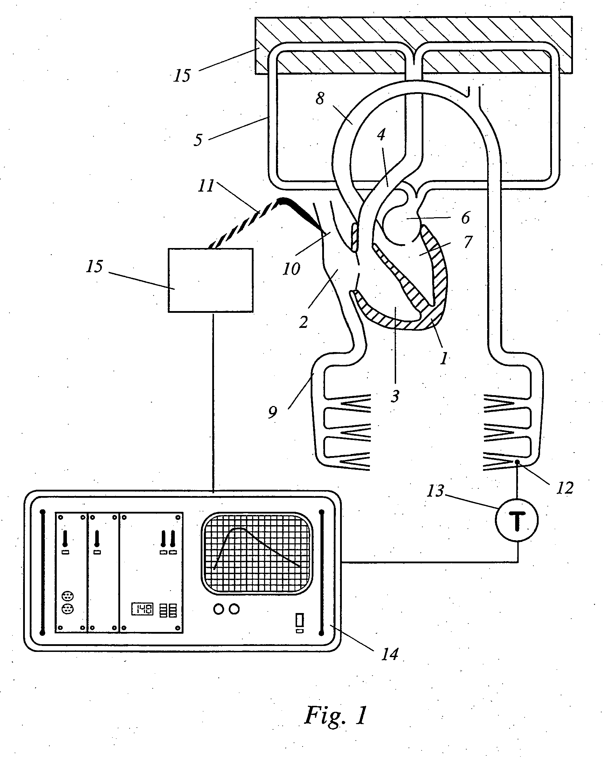

Die in Fig. 1 dargestellte Vorrichtung weist einen Zentralvenenkatheter 11, welcher die Injektion eines gekühlten Bolus in die obere Hohlvene 10 des Patienten gestattet. Um die der Auswertung zugrundeliegende Störfunktion möglichst genau angeben zu können, empfiehlt sich eine möglichst exakte Einhaltung vorgegebener Injektat-Temperatur, Injektat-Menge und (möglichst kurz zu wählender) Injektionsdauer bzw. Erfassung derselben. Mittels einer geeigneten, von einer in die Auswerteeinheit 14 integrierten Steuereinheit gesteuerten Dosierpumpe 15 kann dies auch automatisiert erfolgen. Ebenso ist jedoch auch eine manuell applizierte Bolus-Injektion möglich.

Ein arterieller Katheter 12 (in der Fig. 1 nur durch Markierung des Meßorts angedeutet), welcher einen mit der Auswerteeinheit 14 verbundenen Temperatursensor 13 aufweist, dient der Erfassung eines zeitabhängigen Temperatursignals, aus welchem eine Thermodilutionskurve y gewonnen und als Systemantwort in der programmtechnisch entsprechend ausgestatteten Auswerteeinheit 14 weiterverarbeitet wird.

Der Kälteindikator passiert vom Injektionsort 10 zum Meßort 12 das rechte Atrium 2 und den rechten Ventrikel 3 des Herzens 1, über die Pulmonalarterie 4 den Lungenkreislauf 5 mit extravasalem Thermovolumen (ETV, nährungsweise gleichzusetzen extravasalem Lungenwasservolumen EVLW) 15, das linke Atrium 6, den linken Ventrikel 7 und die Aorta 8.

Anstelle der Applikation eines Kälteindikators können auch andere an sich bekannte Methoden zum Einbringen einer Störung in den Kreislauf vorteilhaft Anwendung finden. Beispielsweise kann ein Wärme-Impuls über den Zentralvenenkatheter 11 eingebracht werden, wofür dieser mit geeigneten Heizmitteln ausgestattet werden kann. Ferner ist auch die Injektion eines optisch detektierbaren Indikators möglich, wobei für die Bestimmung der Systemantwort der arterielle Katheter 12 mit einer faseroptischen Sonde zur Konzentrationsmessung ausgestattet werden kann.

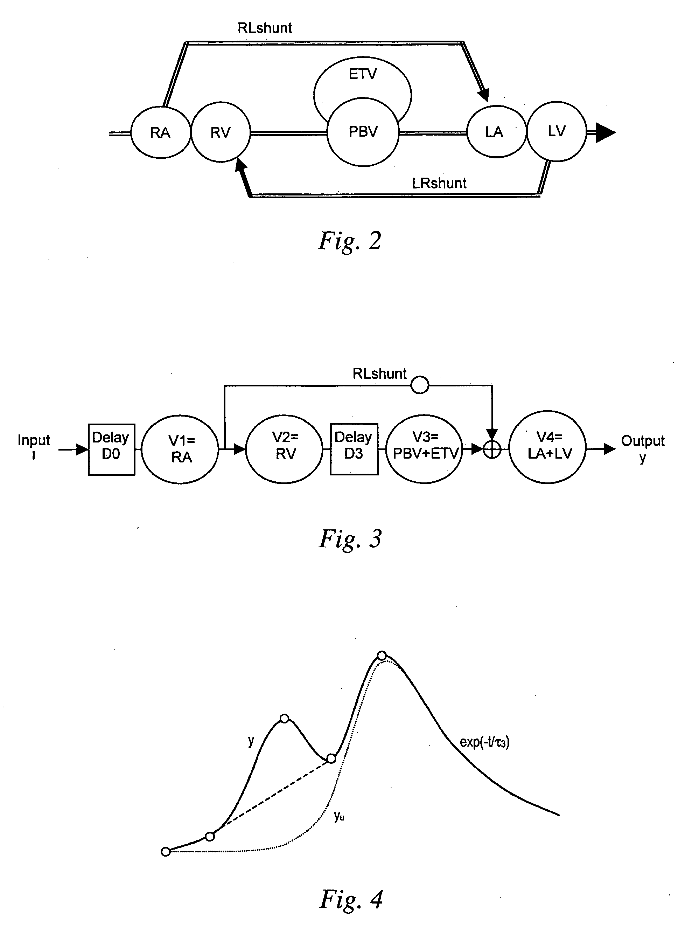

Anhand Fig. 2 sind noch einmal die möglicherweise im Herzen 1 auftretenden Kurzschlußströmungen illustriert: Beim Auftreten von Rechts-Links-Shunt (RLshunt) zwischen dem rechten Atrium RA und dem linken Atrium LA fließt ein Teil des Bluts nicht durch den Lungenkreislauf 5 (in Fig. 2 verdeutlicht durch das pulmonale Blutvolumen PBV mit ebenfalls zu berücksichtigendem extravasalem Thermovolumen ETV) und wird daher nicht oxygeniert, was bei weiteren diagnostischen Betrachtungen von Bedeutung ist. Beim Auftreten von Links-Rechts-Shunt (LRshunt) zwischen dem linken Ventrikel LV und dem rechten Ventrikel RV fließt ein Teil des oxygenierten Bluts nicht in den Körperkreislauf 9 und soll somit nicht dem Herzzeitvolumen CO zugerechnet werden.

Die Berücksichtigung zumindest einer dieser möglichen Kurzschlußströmungen bei der Berechnung des Herzzeitvolumens und/oder anderer hämodynamischer Parameter ist erfindungsgemäß in der programmtechnischen Ausstattung der Auswerteeinheit 14 implementiert.

Fig. 3 illustriert ein erfindungsgemäß für die programmtechnische Ausstattung der Auswerteeinheit 14 geeignetes Modell zur Berücksichtigung des Rechts-Links-Shunts bei der Berechnung hämodynamischer Volumina. Betrachtet wird eine Reihenschaltung aus rechtem Atrium RA, rechtem Ventrikel RV, pulmonalem Blutvolumen PBV (mit ebenfalls zu berücksichtigendem extravasalem Thermovolumen ETV) und Gesamtheit aus linkem Atrium LA und linkem Ventrikel LV. Der Rechts-Links-Shunt ist parallel zu rechtem Ventrikel RV und pulmonalem Blutvolumen PBV geschaltet (vgl. Fig. 2).

Für das rechte Atrium RA wird ein erstes ideal durchmischtes Volumen V1 mit charakteristischer Zeit $\tau 1$, für den rechten Ventrikel ein weiteres ideal durchmischtes Volumen V2 mit charakteristischer Zeit $\tau 2$, für die Gesamtheit aus pulmonalem Blutvolumen PBV und extravasalem Thermovolumen ETV ein drittes ideal durchmischtes Volumen V3 mit charakteristischer Zeit $\tau 3$ und ein Verzögerungsglied ("Delay") D3, sowie für die Gesamtheit aus linkem Atrium LA und linkem Ventrikel LV ein viertes ideal durchmischtes Volumen V4 mit charakteristischer Zeit $\tau 4$ angesetzt. Die cha-rakteristischen Zeiten $\tau n$ sind als Quotient aus dem entsprechenden Volumen Vn und dem Volumenstrom Qn durch dieses Volumen definiert.

Da sich eine lineare Verzögerung in der rechten und der linken Herzhälfte äquivalent auf die Systemantwort ("output") y auswirkt, werden entsprechende Effekte in dem Verzögerungsglied ("Delay") D0 zusammengefaßt. Das Verzögerungsglied D0 kann durch Wahl einer korrigierten Startzeit berücksichtigt werden.

Für die Systemantwort ("Output") y ergibt sich:

$$y = I * D0 * V1 * (s \cdot \delta + (1 - s) \cdot V2 * D3 * V3) * V4$$

mit Faltungsoperator *, Eingangsfunktion ("Input") bzw. Störfunktion I, Dirac-Funktion $\delta$, Shuntverhältnis (Verhältnis von Shunt zu Herzzeitvolumen) $s := RLshunt / CO$.

Die gemessene Dilutionskurve y, deren typischer Verlauf bei Auftreten eines Rechts-Links-Shunts in Fig. 4 skizziert ist, kann in zwei Teile zerlegt werden. Die Flüssigkeitselemente des Bluts, welche den Lungenkreislauf 5 durchlaufen werden durch eine theoretische, kurzschlußfreie Kurve yu beschrieben. Die übrigen Flüssigkeitselemente, welche dem Shunt zuzurechnen sind, werden durch eine theoretische Shunt-Kurve

$$ys = y - yu$$

beschrieben. Das Shuntverhältnis $s = RLshunt / CO$ entspricht dem Quotienten aus dem Integral über die Shunt-Kurve ys und dem Integral über die gemessene Kurve y.

Die Störfunktion I wird als Dirac-Stoßfunktion mit ideal kurzer Injektionszeit und Indikatormenge m betrachtet gemäß

$$I = (m / CO) \cdot \delta(t) = c_0 \cdot \delta(t)$$

Für die Zeitkonstanten gilt

$$\tau_1 = V_1 / CO$$

$$\tau_2 = V_2 / ((1-s) \cdot CO)$$

$$\tau_3 = V_3 / ((1-s) \cdot CO)$$

und

$$\tau_4 = V_4 / CO$$

hieraus ergibt sich

$$y(t) = \frac{c_0 \cdot s}{\tau_1 - \tau_4}\left[ \exp\left(\frac{-t+d_0}{\tau_1}\right) - \exp\left(\frac{-t+d_0}{\tau_4}\right) \right] \cdot \sigma(t-d_0) + y_u(t)$$

$$y_u(t) = \frac{c_0 \cdot (1-s)}{(\tau_1 - \tau_4)(\tau_2 - \tau_3)}\left[ \left(\frac{\tau_2\tau_1}{\tau_2 - \tau_1} - \frac{\tau_2\tau_4}{\tau_2 - \tau_4}\right)\exp\left(\frac{-t+d_0+d_3}{\tau_2}\right) - \left(\frac{\tau_3\tau_1}{\tau_3 - \tau_1} - \frac{\tau_3\tau_4}{\tau_3 - \tau_4}\right)\exp\left(\frac{-t+d_0+d_3}{\tau_3}\right) - \right.$$
$$\left. -\left(\frac{\tau_2\tau_1}{\tau_2 - \tau_1} - \frac{\tau_3\tau_1}{\tau_3 - \tau_1}\right)\exp\left(\frac{-t+d_0+d_3}{\tau_1}\right) + \left(\frac{\tau_2\tau_4}{\tau_2 - \tau_4} - \frac{\tau_3\tau_4}{\tau_3 - \tau_4}\right)\exp\left(\frac{-t+d_0+d_3}{\tau_4}\right) \right] \cdot \sigma(t-d_0-d_3)$$

Darin bezeichnen $d_0$ und $d_3$ die den Verzögerungsgliedern $D_0$ bzw. $D_3$ entsprechenden charakteristischen Zeiten. Die Anfangskonzentration $c_0$ kann durch Integration der Dilutionskurve ermittelt werden:

$$c_0 = \int y\, dt.$$

Für das größte Volumen, V3, wird die charakteristische Zeit $\tau_3$ der Zeitkonstante DST (*down slope time*) des exponentiellen Abfall $y \propto \exp(-t/DST)$ der Dilutionskurve y nach dem Dilutions-Peak gleichgesetzt gemäß

$$\tau_3 = DST.$$

Die gemäß

$$MTT = \int y \cdot t \, dt \, / \int y \, dt$$

aus der Dilutionskurve bestimmbare mittlere Durchgangszeit MTT (*mean transit time*) ist gleich der Summe der charakteristischen Zeiten $\tau 1$, $\tau 2$, $\tau 3$, $d 3$, $\tau 4$, so daß

$$\tau 3 = MTT - DST - \tau 1 - \tau 2 - d 3.$$

Für rechtes und linkes Atrium sowie rechten und linken Ventrikel können vereinfachend konstante Volumenverhältnisse angesetzt werden, beispielsweise

$$\tau 1 = 0{,}6 \cdot \tau 2$$

und

$$\tau 4 = 1{,}3 \cdot \tau 2.$$

Die verbleibenden Modellparameter s, do und d3 können vorzugsweise durch einen Kurvenanpassungsalgorithmus (beispielsweise Levenberg-Marquardt-Algorithmus) bestimmt werden.
Mittels der gemäß obigen Beziehungen ermittelten Modellparameter kann die Auswerteeinheit 14 verschiedene hämodynamische Parameter mit geringeren Fehlerabweichungen berechnen, als dies nach dem Stand der Technik möglich ist:

Herzzeitvolumen:

$$CO = m \, / \, co$$

pulmonales Thermovolumen:

$$PTV = V3 = \tau 3 \cdot (1-s) \cdot CO$$

intrathorakales Thermovolumen:

$$ITTV = V1 + V2 + V3 + V4 = (\tau 1 + \tau 4) \cdot CO + (\tau 2 + \tau 3) \cdot (1-s) \cdot CO$$

globales enddiastolisches Volumen:

$$GEDV = V1 + V2 + V4 = (\tau 1 + \tau 4) \cdot CO + \tau 2 \cdot (1-s) \cdot CO$$

intrathorakales Blutvolumen:

$$ITBV = a \cdot GEDV + b = a \cdot ((\tau_1+\tau_4) \cdot CO + \tau_2 \cdot (1-s) \cdot CO) + b$$

extravasales Lungenwasser:

$$EVLW = ITTV - ITBV = (\tau_1+\tau_4) \cdot CO + (\tau_2+\tau_3) \cdot (1-s) \cdot CO - a \cdot ((\tau_1+\tau_4) \cdot CO + \tau_2 \cdot (1-s) \cdot CO) + b$$

kardialer Funktionsindex:

$$CFI = CO / GEDV = 1 / (\tau_1 + \tau_4 + \tau_2 \cdot (1-s))$$

Die Berechnungsoperationen können vorteilhafterweise im wesentlichen wie folgt in der programmtechnischen Einrichtung der Auswerteeinheit 14 implementiert sein. Nach Abschätzung des Startpunkts der Dilutionskurve y und des Dilutions-Peaks mit geeigneten Kriterien, welche dem Stand der Technik entlehnt sein können, wird die mittlere Durchgangszeit MTT und die Abklingzeit DST berechnet. Mit einem geeigneten Algorithmus wird die Modellfunktion mit geringstmöglicher Abweichung an die meßtechnisch ermittelte Dilutionskurve angepaßt. Mit den Modellparametern aus der angepaßten Modellfunktion werden das Herzzeitvolumen CO und das Shuntverhältnis s berechnet. Anschließend können die Modellvolumina und weitere hämodynamische Parameter berechnet werden.

Sind die Prozessorresourcen der Auswerteeinheit 14 begrenzt, können die Berechnungsoperationen vorteilhafterweise alternativ auch im wesentlichen wie folgt in der programmtechnischen Einrichtung der Auswerteeinheit 14 implementiert sein. Nach Abschätzung des Startpunkts der Dilutionskurve y und des Dilutions-Peaks mit geeigneten Kriterien, welche dem Stand der Technik entlehnt sein können, wird die mittlere Durchgangszeit MTT und die Abklingzeit DST berechnet. Es wird ein vor dem Thermodilutions-Peak liegender Shunt-Peak ermittelt (siehe Fig. 4) sowie eine Tangente (gestrichelte Linie in Fig. 4) an die Dilutionskurve y unterhalb des Kurzschluß-Peaks, welche mit der Dilutionskurve y die größtmögliche Fläche einschließt. Aus charakteristischen Kurvenparametern, beispielsweise dem Startpunkt, dem Maximum des Dilutions-Peaks, dem Maximum des Shunt-Peaks, den Berührpunkten der Tangente der Fläche unter der Dilutionskurve y und der Fläche zwischen der Dilutionskurve y und der Tangente, werden die charakteristischen Modellparameter abgeleitet. Die kurzschlußfreie Kurve yu liegt unter der Tangente. Die Fläche zwischen Tangente und Dilutionskurve y geteilt durch die Fläche unter der Dilutionskurve ergibt einen unteren Näherungswert für den Rechts-Links-Shunt. Weitere Korrekturen und Modellparameter können durch Regression oder Lösen der Modellgleichungen bestimmt werden. Mit den Modellparametern werden das Herzzeitvolumen CO und das Shuntverhältnis s berechnet. Anschließend können die Modellvolumina und weitere hämodynamische Parameter berechnet werden.

Für gewöhnlich kann ein zusätzlicher Peak vor dem Dilutions-Peak stets als Rechts-Links-Shunt betrachtet werden. Ein Rechts-Links-Shunt-Peak kann im Extremfall gar bis etwa 150% höher als der Dilutions-Peak sein.

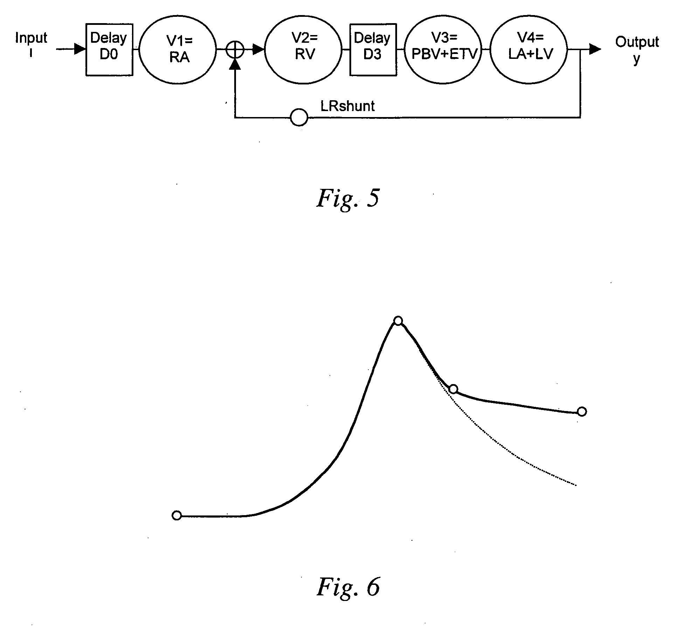

Ein frühzeitiges Ende des exponentiellen Abklingens, wie es in Fig. 6 skizziert ist, nach dem Dilutions-Peak kann als Links-Rechts-Shunt angenommen werden. Dabei ist darauf zu achten, den Links-Rechts-Shunt von einer gewöhnliche Rezirkulation durch den Körperkreislauf 9 zu unterscheiden, welche für gewöhnlich unter 30% des Dilutions-Peaks auftritt.

Zur Berücksichtigung des Links-Rechts-Shunts können vorteilhafterweise prinzipiell ähnliche Berechnungsoperationen in die Auswerteeinheit 14 implementiert sein, wie für die Bestimmung des Rechts-Links-Shunts. Wie in Fig. 5 illustriert wird wiederum eine Reihenschaltung aus rechtem Atrium RA, rechtem Ventrikel RV, pulmonalem Blutvolumen PBV (mit ebenfalls zu berücksichtigendem extravasalem Thermovolumen . ETV) und Gesamtheit aus linkem Atrium LA und linkem Ventrikel LV betrachtet. Der Links-Rechts-Shunt ist in umgekehrter Flußrichtung parallel zu rechtem Ventrikel RV, pulmonalem Blutvolumen PBV und der Gesamtheit aus linkem Atrium LA und linkem Ventrikel LV geschaltet. Zur Vereinfachung können mehrere kleine Volumina durch ein gemeinsames Volumen mit Zeitverzögerung simuliert werden.

Für das rechte Atrium RA wird ein erstes ideal durchmischtes Volumen V1 mit charakteristischer Zeit $\tau_1$, für den rechten Ventrikel ein weiteres ideal durchmischtes Volumen V2 mit charakteristischer Zeit $\tau_2$, für die Gesamtheit aus pulmonalem Blutvolumen PBV und extravasalem Thermovolumen ETV ein drittes ideal durchmischtes Volumen V3 mit charakteristischer Zeit $\tau_3$ und ein Verzögerungsglied ("Delay") D3, sowie für die Gesamtheit aus linkem Atrium LA und linkem Ventrikel LV ein viertes ideal durchmischtes Volumen V4 mit charakteristischer Zeit $\tau_4$ angesetzt. Die charakteristischen Zeiten $\tau_n$ sind als Quotient aus dem entsprechenden Volumen Vn und dem Volumenstrom Qn durch dieses Volumen definiert.

Da sich eine lineare Verzögerung in der rechten und der linken Herzhälfte äquivalent auf die Systemantwort ("Output") y auswirkt, werden entsprechende Effekte in dem Verzögerungsglied ("Delay") D0 zusammengefaßt. Das Verzögerungsglied D0 kann durch Wahl einer korrigierten Startzeit berücksichtigt werden.

Für die Systemantwort ("Output") y ergibt sich:

$$y = ( I * D0 * V1 + y \cdot LRshunt \cdot \delta) * V2 * D3 * V3 * V4$$

mit Faltungsoperator *, Eingangsfunktion ("Input") bzw. Störfunktion I, Dirac-Funktion $\delta$ und Links-Rechts-Shunt LRshunt.

In erster Näherung kann die Stör- bzw. Eingangsfunktion ("Input") I als Dirac-Stoßfunktion $\delta$, d.h. mit verschwindender Dauer betrachtet werden. Üblicherweise dauert eine Injektion jedoch etwa zwei Sekunden. Bei der Shunt-Berechnung kann dies zu einem beachtenswerten Fehler führen. Alternativ besteht daher erfindungsgemäß die Möglichkeit, für die Störfunktion I einen konstanten Fluß 1/p während der Injektionsdauer p anzunehmen, und somit die Störfunktion I als Differenz zweier Heavyside-Sprungfunktionen anzusetzen gemäß

$$I = (\sigma(t) - \sigma(t-p))/p \quad .$$

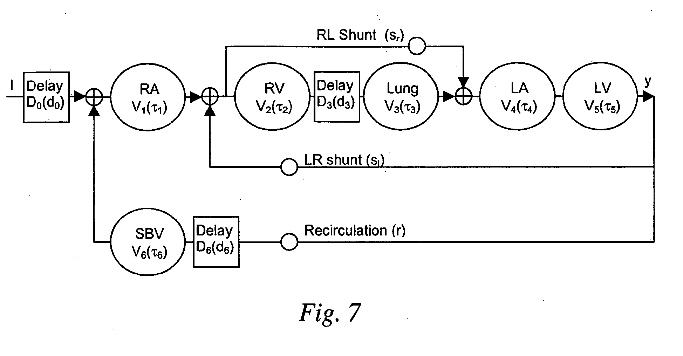

Gemäß einer vorteilhaften Weiterbildung der Erfindung kann mit einem erweiterten Modell und mehrdimensionaler Kurvenanpassung Links-Rechts-Shunt und Rechts-Links-Shunt simultan berücksichtigt und zudem vorzugsweise noch die Rezirkulation durch den Körperkreislauf 9 berücksichtigt werden. Das zugehörige Schaltbild ist in Fig. 7 dargestellt.

Angesetzt werden für das rechte Atrium RA ein erstes ideal durchmischtes Volumen V1 mit charakteristischer Zeit $\tau1$, für den rechten Ventrikel ein weiteres ideal durchmischtes Volumen V2 mit charakteristischer Zeit $\tau2$, für die Gesamtheit aus pulmonalem Blutvolumen PBV und extravasalem Thermovolumen ETV ein drittes ideal durchmischtes Volumen V3 mit charakteristischer Zeit $\tau3$ und ein Verzögerungsglied ("Delay") D3 mit charakteristischer Zeit d3, für das linke Atrium LA ein viertes ideal durchmischtes Volumen V4 mit charakteristischer Zeit $\tau4$, und für den linken Ventrikel LV ein fünftes ideal durchmischtes Volumen V5 mit charakteristischer Zeit $\tau5$. Die charakteristischen Zeiten $\tau n$ sind wiederum als Quotient aus dem entsprechenden Volumen Vn und dem Volumenstrom Qn durch dieses Volumen definiert.

Verzögerungsanteile in der rechten und der linken Herzhälfte werden wiederum Effekte in dem Verzögerungsglied ("Delay") D0 zusammengefaßt, welches durch Wahl einer korrigierten Startzeit berücksichtigt werden kann.

Der Links-Rechts-Shunt mit Shunt-Verhältnis sl ist in umgekehrter Flußrichtung parallel zu rechtem Ventrikel RV, pulmonalem Blutvolumen PBV, linkem Atrium LA und linkem Ventrikel LV geschaltet. Der Rechts-Links-Shunt mit Shunt-Verhältnis sr ist parallel zu rechtem Ventrikel RV und pulmonalem Blutvolumen PBV geschaltet. Betreffend die Rezirkulation r wird ein ideal durchmischtes Volumen V6 mit charakteristischer Zeit $\tau6$ für das systemische Blutvolumen SBV und ein Verzögerungsglied ("Delay") D3 mit charakteristischer Zeit d3 angesetzt.

Für die Systemantwort y ergibt sich:

$$y = ( I * D0 + r \cdot y * D6 * V6) * (V1 + sl \cdot y)*(sr\ \delta + (1 - sr) \cdot D3 * V2 * V3) * V4* V5$$

bzw.

$$y = (1 - sr) \cdot yu + sr \cdot ysr + sl \cdot ysl + r \cdot yr$$

worin die Flüssigkeitselemente des Bluts, welche keinen Kurzschluß durchlaufen durch eine theoretische, kurzschlußfreie Kurve yu, die Flüssigkeitselemente, welche dem Rechts-Links-Shunt zuzurechnen sind, durch eine theoretische Shunt-Kurve ysr, die Flüssigkeitselemente, welche dem Links-Rechts-Shunt zuzurechnen sind, durch eine theoretische Shunt-Kurve ysl, und die Flüssigkeitselemente, welche der Rezirkulation zuzurechnen sind, durch eine theoretische Kurve ysr beschrieben werden.

Die mittlere Durchgangszeit entspricht wieder der Summe der Zeitkonstanten der Reihenschaltung:

$$MTT = \tau1 + \tau2 + d3 + \tau3 + \tau4 + \tau5$$

Wenn alle Volumina des Herzens gleich Vh gesetzt werden und nur der erste Rezirkulationsdurchgang berücksichtigt wird, ergibt sich:

$$Vh = V1 = V2 = V4 = V5$$

$$yu = (1 - sr) \cdot I * D0 * D3 * Vh^4 * V3$$

$$ysr = sr \cdot I * D0 * Vh^3$$

$$ysl \approx sl \cdot (yu + ysr) * D3 * Vh^3 * V3$$

$$yr \approx r \cdot (yu + ysr) * D6 * V6 * D3 * Vh^4 * V3$$

und schließlich

$$y_u(t) = (1-s)\frac{\sigma(t)-\sigma(t-p)}{p} * \left[\frac{t^3}{6\tau_h^{\ 4}} \cdot \exp\left(\frac{-t}{\tau_h}\right)\right] * \left[\frac{1}{\tau_3} \cdot \exp\left(\frac{-t}{\tau_3}\right)\right] * \delta(t - d_0 - d_3)$$

$$y_{sr}(t) = s\frac{\sigma(t)-\sigma(t-p)}{p} * \left[\frac{t^2}{2\tau_h^{\ 3}} \cdot \exp\left(\frac{-t}{\tau_h}\right)\right] * \delta(t - d_0)$$

$$y_{sl}(t) \approx s_l(y_u(t) + y_{sr}(t)) * \left[\frac{t^2}{2\tau_h^{\ 3}} \cdot \exp\left(\frac{-t}{\tau_h}\right)\right] * \left[\frac{1}{\tau_3} \cdot \exp\left(\frac{-t}{\tau_3}\right)\right] * \delta(t - d_3)$$

$$y_r(t) \approx r(y_u(t) + y_{sr}(t)) * \left[\frac{1}{\tau_6} \cdot \exp\left(\frac{-t}{\tau_6}\right)\right] * \left[\frac{t^3}{6\tau_h^{\ 4}} \cdot \exp\left(\frac{-t}{\tau_h}\right)\right] * \left[\frac{1}{\tau_3} \cdot \exp\left(\frac{-t}{\tau_3}\right)\right] * \delta(t - d_3 - d_6)$$

Allgemein können, wie oben erwähnt, auch nicht-diffusible intravasale Indikatoren, wie z.B. LiCl oder ICG verwendet werden. Bei Verwenung nicht-diffusibler intravasaler Indikatoren können Herzzeitvolumen (*Cardiac Output - CO*) und globales enddiastolisches Volumen (GEDV) bestimmt werden, nicht jedoch extravasales Lungenwasser (EVLW). Die Algorithmen können dabei prinzipiell gegenüber den obenbeschriebenen Algorithmen unverändert bleiben mit der Ausnahme, daß dann das größte intrathorakale Ausbreitungsvolumen dem intrathorakalen Blutvolumen ITBV (bei LiCl- oder ICG-Indikator) anstatt dem intrathorakalen Thermovolumen ITTV (bei Kälteindikator) entspricht.

**Patentansprüche**

1. Vorrichtung zur Bestimmung mindestens eines hämodynamischen Parameters eines Lebewesens, aufweisend

   - eine extrakardiale Wirkeinheit mit Mitteln zum Einbringen einer durch eine Störfunktion I charakterisierbaren Störung in das Blutkreislaufsystem des Lebewesens mittels definierten Einwirkens auf venöses Blut,
   - eine Sensorvorrichtung (13) zur Erzeugung eines Messsignals in Abhängigkeit einer physikalischen Größe arteriellen Bluts, welche eine durch die Störfunktion bedingte Systemantwort des Blutkreislaufsystems charakterisiert, und
   - eine Auswerteeinheit (14), welche einen Eingangskanal zum fortlaufenden Einlesen des Messsignals aufweist,

   wobei die Auswerteeinheit (14) programmtechnisch dazu eingerichtet ist, den hämodynamischen Parameter aus einer dem zeitlichen Verlauf des Messsignals entsprechenden Dilutionskurve y zu berechnen und hierbei eine mögliche Kurzschlussströmung von der rechten zur linken Herzhälfte (RL-Shunt) und/oder von der linken zur rechten Herzhälfte (LR-Shunt des Lebewesens zu berücksichtigen, **dadurch gekennzeichnet, dass** progammtechnisch zum Berechnen des hämodynamischen Parameters ein Modell für Herz und Lungenkreislauf des Lebewesens zugrundegelegt ist, welches eine Reihenschaltung mehrerer Systemglieder und mindestens ein parallelgeschaltetes Systemglied zur Berücksichtigung der Kuzschlussströmung umfasst, und die programmtechnische Einrichtung der Auswerteeinheit (14) Berechnungsoperationen umfasst, welche von einer diesem Modell entsprechenden Modellfunktion, die die mathematische Faltung von Ausdrücken umfasst, welche die in Reihe geschalteten Systemglieder und die Störfunktion verkörpern, für die Systemantwort herleitbar sind,

   wobei die Ausdrücke jeweils eine charakteristische Zeit als Modellparameter aufweisen, und in dem zugrundegelegten Modell

   - für das rechte Atrium RA (2) des Lebewesens ein erstes ideal durchmischtes Volumen,
   - für den rechten Ventrikel RV (3) des Lebewesens ein zweites ideal durchmischtes Volumen,
   - für die Gesamtheit aus pulmonalem Blutvolumen PBV und extravasalem Thermovolumen ETV des Lebewesens ein drittes ideal durchmischtes Volumen und ein Verzögerungsglied,
   - sowie für die Gesamtheit aus linkem Atrium LA (6) und linkem Ventrikel LV (7) des Lebewesens zumindest ein viertes ideal durchmischtes Volumen als Systemglieder der Reihenschaltung angesetzt sind.

2. Vorrichtung gemäß Anspruch 1, wobei die programmtechnische Einrichtung der Auswerteeinheit (14) Operationen zur Berechnung mindestens einer der folgenden Größen als hämodynamischen Parameter aufweist:

   - Herzzeitvolumen CO,
   - pulmonales Thermovolumen PTV,
   - intrathorakales Thermovolumen ITTV,
   - globales enddiastolisches Volumen GEDV,
   - intrathorakales Blutvolumen ITBV,
   - extravasales Lungenwasser EVLW,
   - kardialer Funktionsindex CFI.

3. Vorrichtung gemäß einem der vorangehenden Ansprüche, wobei die programmtechnische Einrichtung der Auswerteeinheit (14) Operationen für die Abschätzung eines Startpunkts der Dilutionskurve y und Operationen für die Bestimmung einer mittleren Durchgangszeit MTT gemäß

$$MTT = \int y \cdot t \, dt \, / \int y \, dt$$

mit der Zeit t aus der Dilutionskurve y aufweist,
und wobei in der programmtechnischen Einrichtung die mittleren Durchgangszeit MTT als Summe der charakteristischen Zeiten der ersten, zweiten, dritten und vierten ideal durchmischtes Volumina sowie des Verzögerungsglieds berücksichtigt ist.

4. Vorrichtung gemäß einem der vorangehenden Ansprüche, wobei die programmtechnische Einrichtung der Auswerteinheit (14) Operationen für die Erkennung eines Dilutions-Peaks und Operationen für die Bestimmung einer exponentiellen Abklingzeit DST aus einem exponentiellen Abfall der Dilutionskurve y nach dem Dilutions-Peak gemäß

$$y \propto \exp(-t/DST)$$

mit der Zeit t aufweist,
und wobei in der programmtechnischen Einrichtung die exponentielle Abklingzeit DST als charakteristische Zeit des dritten ideal durchmischten Volumens berücksichtigt ist.

5. Vorrichtung gemäß einem der vorangehenden Ansprüche, wobei in der programmtechnischen Einrichtung der Auswerteeinheit (14) ein konstantes Verhältnis der charakteristischen Zeiten der ersten, zweiten und vierten ideal durchmischten Volumina zueinander vorgesehen ist.

6. Vorrichtung gemäß einem der vorangehenden Ansprüche, wobei die programmtechnische Einrichtung der Auswerteinheit (14) Operationen zur Ermittlung der charakteristischen Zeiten durch rechnerische Anpassung einer der Modellfunktion entsprechenden Modellkurve an die Dilutionskurve y aufweist.

7. Vorrichtung gemäß Anspruch 6, wobei die rechnerische Anpassung der der Modellfunktion entsprechenden Modellkurve an die Dilutionskurve y mittels eines Levenberg-Marquardt-Algorithmus implementiert ist.

8. Vorrichtung gemäß einem der vorangehenden Ansprüche, wobei die programmtechnische Einrichtung der Auswerteinheit (14) Operationen für die Ermittlung eines Kurzschluss-Peaks als zusätzlichem Peak der Dilutionskurve y vor dem Dilutions-Peak aufweist, und der Fall der Nichtexistenz eines Kurzschluss-Peaks als Bedingung für die Nichtexistenz einer Kurzschlussströmung vom rechten Atrium (2) zum linken Ventrikel (7) des Lebewesens vorgesehen ist.

9. Vorrichtung gemäß Anspruch 8, wobei die programmtechnische Einrichtung der Auswerteeinheit (14) Operationen zum Bestimmen einer Tangente an die Dilutionskurve y unterhalb des Kurzschluss-Peaks, welche mit der Dilutionskurve y die größtmögliche Fläche einschließt, aufweist.

10. Vorrichtung gemäß einem der vorangehenden Ansprüche, wobei die programmtechnische Einrichtung der Auswerteinheit (14) Operationen für die Ermittlung des Werts der Dilutionskurve y am Endes eines exponentiellen Abfalls der Dilutionskurve y nach dem Dilutions-Peak aufweist,
und der Fall, dass der ermittelte Wert einen vorgegebenen Anteil des Werts der Dilutionskurve y am Dilutionspeak übersteigt, als Bedingung für die Existenz einer Kurzschlussströmung vom linken Ventrikel zum rechten Ventrikel des Lebewesens vorgesehen ist.

11. Vorrichtung gemäß Anspruch 10, wobei der vorgegebenen Anteil mindestens 30 Prozent ist.

12. Vorrichtung gemäß einem der vorangehenden Ansprüche, wobei in der programmtechnischen Einrichtung der Auswerteeinheit (14) für die Störfunktion eine Stoßfunktion der Form

$$I = co\ \delta(t),$$

worin δ die Dirac-Stoßfunktion und co ein Koeffizient ist, angesetzt ist.

13. Vorrichtung gemäß Anspruch 12, wobei der Koeffizient co als Quotient aus einer Indikatorquantität m und einem Herzzeitvolumen CO des Lebewesens gemäß

$$co = m/CO$$

angesetzt ist.

14. Vorrichtung gemäß einem der Ansprüche 12-13, wobei die programmtechnische Einrichtung der Auswerteeinheit (14) Operationen zur Bestimmung des Koeffizienten co als Integral der Dilutionskurve y über die Zeit t gemäß

$$co = \int y\, dt$$

aufweist.

15. Vorrichtung gemäß einem der vorangehenden Ansprüche, wobei für die Störfunktion die Differenz zweier Sprung-funktionen gemäß

$$I = (1/p) \cdot [\sigma(t) - \sigma(t-p)]\,,$$

worin σ die Heavyside-Sprungfunktion und p die Dauer des definierten Einwirkens auf zentralvenöses Blut ist, angesetzt ist.

16. Vorrichtung gemäß einem der vorangehenden Ansprüche, wobei extrakardiale Wirkeinheit eine zentralvenöse Kathetereinheit (11) ist.

17. Vorrichtung gemäß einem der vorangehenden Ansprüche, aufweisend eine arterielle Kathetereinheit (12), welche mit der Sensorvorrichtung (13) ausgerüstet ist.

18. Vorrichtung gemäß einem der vorangehenden Ansprüche, wobei die Mittel zum Einbringen der Störung Mittel zum Herbeiführen einer Temperaturänderung venösen Bluts sind, und die die Systemantwort des Blutkreislaufsystems charakterisierende physikalische Größe eine Temperatur arteriellen Bluts ist.

19. Vorrichtung gemäß Anspruch 18, wobei die Mittel zum Einbringen der Störung Heizmittel zum Abgeben eines Heizimpulses an zentralvenöses Blut umfassen.

20. Vorrichtung gemäß Anspruch 18, wobei die Mittel zum Einbringen einer Störung in das Blutkreislaufsystem Mittel zum Injizieren eines gekühlten Bolus in venöses Blut umfassen.

21. Vorrichtung gemäß einem der Ansprüche 1-17, wobei die Mittel zum Einbringen einer Störung Mittel zum Injizieren eines Indikators in venöses Blut umfassen, und die die Systemantwort des Blutkreislaufsystems charakterisierende physikalische Größe eine Indikatorkonzentration in arteriellem Blut ist.

22. Vorrichtung gemäß einem der vorangehenden Ansprüche, ferner aufweisend eine Steuereinheit zum Steuern der Mittel zum Einbringen einer Störung in das Blutkreislaufsystem.

**Claims**

1. Device for determining at least one hemodynamic parameter of a living being, which has the following:

  - an extracardial effect unit having means for introducing a disruption that can be **characterized by** means of a disruption function I into the cardiovascular system of the living being, by means of a defined effect on venous blood,
  - a sensor device (13) for producing a measurement signal as a function of a physical variable of arterial blood, which characterizes a system response of the cardiovascular system brought about by the disruption function, and
  - an evaluation unit (14) that has an input channel for continuously reading in the measurement signal,

wherein the evaluation unit (14) is set up, in terms of program technology, to calculate the hemodynamic parameter from a dilution curve y that corresponds to the time progression of the measurement signal and, in this connection, to take into consideration a possible short-circuit current from the right to the left half of the heart (RL shunt) and/or from the left to the right half of the heart (LR shunt) of the living being,

**characterized in that** for calculating the hemodynamic parameter, a model for the cardiovascular and pulmonary circulation system of the living being is used as the basis that comprises a serial circuit of several system elements and at least one system element connected in parallel, for taking the short-circuit current into consideration, and the program technology set-up of the evaluation unit (14) comprises calculation operations that can be derived from a model function for the system response that corresponds to this model and that comprises the mathematical convolution of terms that incorporate the system elements connected in series and the disruption function,

wherein the terms each have a characteristic time as a model parameter, and in the underlying model,

- a first ideally mixed volume for the right atrium RA (2) of the living being,
- a second ideally mixed volume for the right ventricle RV (3) of the living being,
- a third ideally mixed volume for the totality of pulmonary blood volume PBV and extravasal thermal volume ETV of the living being and a delay element, and
- at least a fourth ideally mixed volume for the totality of the left atrium (6) and the left ventricle LV (7) of the living being

are assumed as system elements of the serial circuit.

2. Device according to claim 1, wherein the program technology set-up of the evaluation unit (14) has operations for calculating at least one of the following variables as a hemodynamic parameter:

- cardiac output CO,
- pulmonary thermal volume PTV,
- intrathoracic thermal volume ITTV,
- global end-diastolic volume GEDV,
- intrathoracic blood volume ITBV,
- extravasal lung water EVLW,
- cardiac function index CFI.

3. Device according to any of the preceding claims, wherein the program technology set-up of the evaluation unit (14) has operations for estimating a starting point of the dilution curve y and operations for determining a mean transit time MTT according to

$$MTT = \int y \cdot t \, dt / \int y \, dt$$

with the time t from the dilution curve y,
and wherein the mean transit time MTT is taken into consideration in the program technology set-up as the sum of the characteristic times of the first, second, third, and fourth ideally mixed volumes as well as of the delay element.

4. Device according to any of the preceding claims, wherein the program technology set-up of the evaluation unit (14) has operations for recognizing a dilution peak and operations for determining an exponential decay time DST from an exponential drop of the dilution curve y after the dilution peak, according to

$$y \propto \exp(-t/DST)$$

with the time t,
and wherein the exponential decay time DST is taken into consideration in the program technology set-up as a characteristic time of the third ideally mixed volume.

5. Device according to any of the preceding claims, wherein a constant ratio of the characteristic times of the first, second, and fourth ideally mixed volumes relative to one another is provided in the program technology set-up of the evaluation unit (14).

6. Device according to any of the preceding claims, wherein the program technology set-up of the evaluation unit (14) has operations for determining the characteristic times by means of fitting, by calculations, of a model curve that corresponds to one of the model functions to the dilution curve y.

7. Device according to claim 6, wherein the fitting, by calculations, of the model curve corresponding to the model function to the dilution curve y is implemented by means of a Levenberg-Marquardt algorithm.

8. Device according to any of the preceding claims, wherein the program technology set-up of the evaluation unit (14) has operations for determining a short-circuit peak as an additional peak of the dilution curve y ahead of the dilution peak, and the case of the non-existence of a short-circuit peak is provided as a condition for the non-existence of a short-circuit current from the right atrium (2) to the left ventricle (7) of the living being.

9. Device according to claim 8, wherein the program technology set-up of the evaluation unit (14) has operations for determining a tangent to the dilution curve y below the short-circuit peak, which encloses the greatest possible area with the dilution curve y.

10. Device according to any of the preceding claims, wherein the program technology set-up of the evaluation unit (14) has operations for determining the value of the dilution curve y at the end of an exponential drop of the dilution curve y after the dilution peak,
and the case that the value determined exceeds a predetermined proportion of the value of the dilution curve y at the dilution peak is provided as a condition for the existence of a short-circuit current from the left ventricle to the right ventricle of the living being.

11. Device according to claim 10, wherein the predetermined proportion is at least 30 percent.

12. Device according to any of the preceding claims, wherein a Dirac function having the form

$$I = co\,\delta(t),$$

is assumed for the disruption function in the program technology set-up of the evaluation unit (14), wherein $\delta$ is the Dirac delta function and co is a coefficient.

13. Device according to claim 12, wherein the coefficient co is assumed to be a quotient of an indicator quantity m and a cardiac output CO of the living being according to

$$co = m/CO.$$

14. Device according to any of claims 12-13, wherein the program technology set-up of the evaluation unit (14) has operations for determining the coefficient co as an integral of the dilution curve y over the time t, according to

$$co = \int y\,dt$$

15. Device according to any of the preceding claims, wherein the difference between two step functions is assumed for the disruption function, according to

$$I = (1/p)\cdot[\sigma(t)-\sigma(t-p)],$$

wherein σ is the Heaviside step function and p is the duration of the defined effect on central vein blood.

16. Device according to any of the preceding claims, wherein the extracardial effect unit is a central venous catheter unit (11).

17. Device according to one of the preceding claims, comprising an arterial catheter unit (12) that is equipped with the sensor device (13).

18. Device according to one of the preceding claims, wherein the means for introducing the disruption are means for bringing about a temperature change in venous blood, and the physical variable that characterizes the system response of the blood circulation is a temperature of arterial blood.

19. Device according to claim 18, wherein the means for introducing the disruption comprise heating means for giving off a temperature impulse to central-venous blood.

20. Device according to claim 18, wherein the means for introducing a disruption in the blood circulation comprise means for injecting a cooled bolus into venous blood.

21. Device according to any of claims 1-17, wherein the means for introducing a disruption comprise means for injecting an indicator into venous blood, and the physical variable that characterizes the system response of the blood circulation is an indicator concentration in arterial blood.

22. Device according to any of the preceding claims, furthermore comprising a control unit for controlling the means for introducing a disruption into the blood circulation.

**Revendications**

1. Dispositif destiné à déterminer au moins un paramètre hémodynamique d'un être vivant, comprenant :

   - une entité active extracardiaque, avec un moyen destiné à introduire dans le système cardiovasculaire de l'être vivant une perturbation pouvant être **caractérisée par** une fonction perturbatrice I par une action définie sur le sang veineux ;
   - un dispositif capteur (13), destiné à produire un signal de mesure en fonction d'une grandeur physique du sans artériel qui est **caractérisée par** une réponse systémique du système cardiovasculaire à la fonction perturbatrice; et
   - une unité d'analyse (14) qui possède un canal d'entrée afin de lire le signal de mesure en continu ;

   dans lequel l'unité d'analyse (14) est conçue au plan de la programmation pour calculer le paramètre hémodynamique à partir d'une courbe de dilution y correspondant à l'allure du signal de mesure dans le temps tout en tenant compte d'un écoulement en court-circuit entre les moitiés droite et gauche (shunt RL) et/ou entre les moitiés gauche et droite (shunt LR) de l'être vivant ;
   **caractérisé par le fait qu'**est pris comme base au plan de la programmation, pour le calcul du paramètre hémodynamique, un modèle du coeur et du circuit pulmonaire de l'être vivant qui comprend un circuit en série de plusieurs organes du système et au moins un organe du système disposé en parallèle pour tenir compte de l'écoulement en court-circuit et **par le fait que** la conception de la programmation de l'unité d'analyse (14) comprend des opérations de calcul qui peuvent être dérivées pour la réponse systémique d'une fonction de modèle qui correspond à ce modèle et qui comprend la traduction mathématique d'expressions qui incarnent les organes disposés en série et la fonction perturbatrice ;
   dans lequel les expressions possèdent chacune un instant caractéristique comme paramètre du modèle et dans lequel on retient dans le modèle de base, comme organes du circuit en série,

   - un premier volume mélangé idéalement pour l'oreillette droite RA (2) de l'être vivant ;
   - un deuxième volume mélangé idéalement pour le ventricule droit (RV) (3) de l'être vivant ;
   - un troisième volume mélangé idéalement et un organe de temporisation pour l'ensemble du volume sanguin pulmonaire PBV et le thermovolume extravasculaire ETV de l'être vivant ; ainsi qu'
   - au moins un quatrième volume mélangé idéalement pour l'ensemble de l'oreillette gauche LA (6) et du ventricule gauche LV (7) de l'être vivant.

**2.** Dispositif selon la revendication 1, dans lequel la conception de la programmation de l'unité d'analyse (14) comprend des opérations pour le calcul d'au moins l'une des grandeurs suivantes comme paramètre hémodynamique :

- le débit cardiaque CO ;
- le thermovolume pulmonaire PTV ;
- le thermovolume intrathoracique ITTV ;
- le volume global télédiastolique GEDV ;
- le volume sanguin intrathoracique ITBV ;
- le liquide pulmonaire extravasculaire EVLW ;
- l'indice fonctionnel cardiaque CFI.

**3.** Dispositif selon l'une des revendications précédentes, dans lequel la conception de la programmation de l'unité d'analyse (14) comprend des opérations pour l'estimation d'un point de départ de la courbe de dilution y et des opérations pour la détermination d'un temps moyen de transit MTT à l'aide de l'équation :

$$\mathrm{MTT} = \int y \cdot t \ dt \ / \ \int y \ dt$$

avec le temps t à partir de la courbe de dilution y et dans lequel, dans la conception de la programmation, le temps moyen de transit MTT est pris comme la somme des temps caractéristiques des premier, deuxième, troisième et quatrième volumes mélangés idéalement et de l'organe de temporisation.

**4.** Dispositif selon l'une des revendications précédentes, dans lequel la conception de la programmation de l'unité d'analyse (14) comprend des opérations pour la détection d'un pic de dilution et des opérations pour la détermination d'un temps de décroissance exponentielle DST à partir de la chute exponentielle de la courbe de dilution y après le pic de dilution, à l'aide de l'équation :

$$y \propto \exp(-t/\mathrm{DST})$$

avec le temps t
et dans lequel, dans la conception de la programmation, le temps de décroissance exponentielle DST est pris comme un temps caractéristique du troisième volume mélangé idéalement.

**5.** Dispositif selon l'une des revendications précédentes, dans lequel, dans la conception de la programmation de l'unité d'analyse (14), est prévu un rapport constant des temps caractéristiques des premier, deuxième et quatrième volumes mélangés idéalement entre eux.

**6.** Dispositif selon l'une des revendications précédentes, dans lequel la conception de la programmation de l'unité d'analyse (14) comprend des opérations de détermination des temps caractéristiques par un calcul de l'adaptation à la courbe de dilution y de l'une des courbes de modèle correspondant à la fonction de modèle.

**7.** Dispositif selon la revendication 6, dans lequel le calcul de l'adaptation à la courbe de dilution y de celle des courbes de modèle correspondant à la fonction de modèle est mis en oeuvre à l'aide d'un algorithme de Levenberg-Marquardt.

**8.** Dispositif selon l'une des revendications précédentes, dans lequel la conception de la programmation de l'unité d'analyse (14) comprend des opérations pour la détermination d'un pic de court-circuit comme pic supplémentaire de la courbe de dilution y avant le pic de dilution, et dans lequel la situation de l'inexistence d'un pic de court-circuit est prévue comme condition de l'inexistence de l'écoulement en court-circuit entre l'oreillette droite (2) et le ventricule gauche (7) de l'être vivant.

**9.** Dispositif selon la revendication 8, dans lequel la conception de la programmation de l'unité d'analyse (14) comprend des opérations pour la détermination d'une tangente à la courbe de dilution y située au-dessous du pic de court-circuit et incluant avec la courbe de dilution y la surface aussi grande que possible.

**10.** Dispositif selon l'une des revendications précédentes, dans lequel la conception de la programmation de l'unité d'analyse (14) comprend des opérations pour la détermination de la valeur de la courbe de dilution y à la fin d'une chute exponentielle de la courbe de dilution y après le pic de dilution
et dans lequel la situation dans laquelle la valeur déterminée dépasse une proportion prédéterminée de la valeur de la courbe de dilution y au pic de dilution est prévue comme une condition de l'existence d'un écoulement en court-circuit entre le ventricule gauche et le ventricule droit de l'être vivant.

**11.** Dispositif selon la revendication 10, dans lequel la proportion prédéterminée est d'au moins 30 pour cent.

**12.** Dispositif selon l'une des revendications précédentes, dans lequel, dans la conception de la programmation de l'unité d'analyse (14), on utilise pour la fonction perturbatrice une fonction impulsionnelle de la forme :

$$l = co\ \delta(t)$$

où $\delta$ est la fonction impulsionnelle de Dirac et co est un coefficient.

**13.** Dispositif selon la revendication 12, dans lequel le coefficient co est le quotient d'une quantité indicatrice m par le débit cardiaque CO de l'être vivant, selon l'équation :

$$co = m/CO.$$

**14.** Dispositif selon l'une des revendications 12 et 13, dans lequel la conception de la programmation de l'unité d'analyse (14) comprend des opérations pour la détermination du coefficient co comme une intégrale de la courbe de dilution y sur le temps t, selon l'équation :

$$co = \int y\ dt.$$

**15.** Dispositif selon l'une des revendications précédentes, dans lequel on pose comme fonction perturbatrice la différence de deux fonctions échelon selon :

$$l = (1/p)\ \cdot\ [\sigma(t) - \sigma(t-p)],$$

où $\sigma$ est la fonction échelon de Heavyside et p la durée de l'action définie sur le sang veineux central.

**16.** Dispositif selon l'une des revendications précédentes, dans lequel l'entité active extracardiaque est une unité de cathéter veineux central (11).

**17.** Dispositif selon l'une des revendications précédentes, comprenant une unité de cathéter artériel (12) qui est dotée du dispositif capteur (13).

**18.** Dispositif selon l'une des revendications précédentes, dans lequel le moyen d'introduction de la perturbation est un moyen de provoquer une variation de la température du sang veineux et la grandeur physique qui caractérise la réponse systémique du système cardiovasculaire est une température du sang artériel.

**19.** Dispositif selon la revendication 18, dans lequel le moyen d'introduction de la perturbation comprend un moyen de chauffage afin d'appliquer une impulsion thermique au sang veineux central.

**20.** Dispositif selon la revendication 18, dans lequel le moyen d'introduction d'une perturbation dans le système cardiovasculaire est un moyen d'injection d'un bolus refroidi dans le sang veineux.

**21.** Dispositif selon l'une des revendications 1 à 17, dans lequel le moyen d'introduction d'une perturbation comprend un moyen d'injection d'un indicateur dans le sang veineux et la grandeur physique caractéristique de la réponse systémique du système cardiovasculaire est une concentration de l'indicateur dans le sang artériel.

**22.** Dispositif selon l'une des revendications précédentes, comprenant en outre une unité de commande destinée à commander le moyen d'introduction d'une perturbation dans le système cardiovasculaire.

Fig. 1

Fig. 2

Fig. 3

Fig. 4

*Fig. 5*

*Fig. 6*

*Fig. 7*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9321823 A1
- WO 0130237 A1
- US 5595181 A

**In der Beschreibung aufgeführte Nicht-Patentliteratur**

- **J. K. G. Wietasch.** Die Doppelindikatordilution zur Quantifizierung von Herzzeitvolumen und Links-Rechts-Shunt bei Patienten mit kongenitalem Vitium cordis. *Göttingen,* 1995